(19) Europäisches Patentamt
European Patent Office
Office européen des brevets

(11) EP 1 262 186 B1

(12) EUROPEAN PATENT SPECIFICATION

(45) Date of publication and mention of the grant of the patent:
**21.04.2004 Bulletin 2004/17**

(51) Int Cl.[7]: **A61K 31/7052**, A61K 47/10, A61K 9/08

(21) Application number: **02253796.3**

(22) Date of filing: **30.05.2002**

(54) **Azalide antibiotic compositions**

Antibiotische Azalid-Zusammensetzungen

Compositions antibiotiques à base d'azalide

(84) Designated Contracting States:
**AT BE CH CY DE DK ES FI FR GB GR IE IT LI LU MC NL PT SE TR**

(30) Priority: **31.05.2001 US 294677 P**

(43) Date of publication of application:
**04.12.2002 Bulletin 2002/49**

(73) Proprietor: **Pfizer Products Inc.**
**Groton, Connecticut 06340 (US)**

(72) Inventor: **Boettner, Wayne A.,**
**Pfizer Global Research and Dev**
**Groton, Connecticut 06340 (US)**

(74) Representative: **Motion, Keith Robert et al**
**Pfizer Limited**
**Patents Department**
**Ramsgate Road**
**Sandwich, Kent CT13 9NJ (GB)**

(56) References cited:
**WO-A-01/55158**
**WO-A-01/81358**
**WO-A-98/56801**
**US-B1- 6 239 112**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 262 186 B1

Printed by Jouve, 75001 PARIS (FR)

## Description

This invention relates to aqueous compositions comprising an azalide antibiotic compound, a non-aqueous solvent and one or more acids, and to methods for preparing them. This invention further relates to pharmaceutical compositions comprising an azalide antibiotic compound, a non-aqueous solvent and one or more acids, and to methods for treating a mammal comprising administering to a mammal in need of such treatment a composition of the invention.

Macrolide antibiotic agents active against a wide variety of bacterial and protozoa infections in mammals, fish and birds are well-known. These compounds generally have a macrocyclic lactone ring of 12 to 22 carbon atoms to which one or more sugar moieties are attached. Macrolide antibiotics act on the 50S ribosomal subunit to inhibit protein synthesis in microorganisms. Examples of macrolide antibiotics include azithromycin, which is a derivative of erythromycin A, and other azalide compounds, for example 8a-N-azalides and 9a-N-azalides such as those disclosed in, e. g., International Patent Publications WO 98/56801 and WO 99/12552, and European patent application EP 508699 A1.

Azalide compounds as described in the art are currently referred to as "triamilide" compounds; in this application, applicants have used the old "azalide" terminology, but it is to be understood that the present invention is applicable to compounds referred to in the art under the new "triamilide" terminology.

Development of aqueous compositions containing azalide compounds as the active ingredient has presented significant challenges. For example, the lactone ring and sugars of azalides are easily hydrolyzed in even mildly acidic or mildly basic pH environments, decreasing the potency and shelf-life of an antibiotic composition.

Accordingly, it is an object of the present invention to provide antibiotic compositions, and methods for preparing them, that overcome the above-mentioned disadvantages.

## Summary of the Invention

The present invention relates to an aqueous composition comprising:

(a) a compound of formula 1

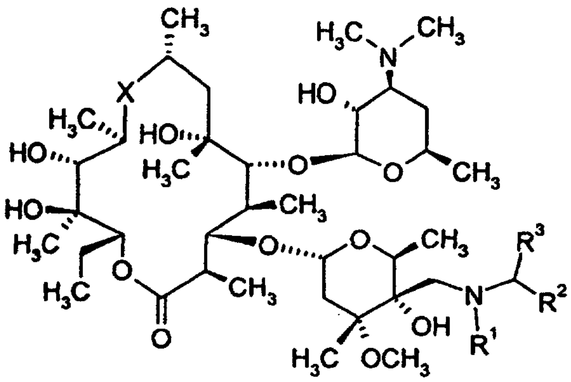

1;

(b) propylene glycol in an amount of from 25% to 75% by weight relative to the total volume;
(c) one or more acids to provide a pH of the composition of from 4.5 to 6.5; and
(d) water; wherein

$R^1$ and $R^2$ are each independently selected from the group consisting of hydrogen, $(C_1-C_{10})$alkyl, and $(C_3-C_7)$cycloalkyl; and
$R^3$ is selected from the group consisting of hydrogen and $(C_1-C_{10})$alkyl; and
X is $-N(R^4)CH_2-$ or $-CH_2N(R^4)-$, wherein $R^4$ is $(C_1-C_3)$alkyl.

The term "azalide composition" as used in this application, unless otherwise specified, is used throughout this application to refer to any composition comprising a compound of formula 1, propylene glycol in an amount of from 25% to 75% by weight relative to the total volume, and one or more acids to provide a pH of the composition of from 4.5 to 6.5; wherein $R^1$ to $R^4$ and X are as defined above. The term "azalide composition" as used herein includes each of the embodiments, preferred, more preferred and particularly preferred embodiments described herein.

As used in this application, the terms "alkyl" and "cycloalkyl" include saturated alkyl groups having both straight

or branched chains, e.g., n-propyl and isopropyl, n-butyl, isobutyl and *tert*-butyl.

In an embodiment, $R^2$ is methyl. In a preferred embodiment, either $R^1$ or $R^3$ is hydrogen and the other is methyl, and $R^2$ is methyl. In a particularly preferred embodiment, $R^1$ is hydrogen, $R^2$ is methyl and $R^3$ is methyl. In another particularly preferred embodiment, $R^1$ is methyl, $R^2$ is methyl and $R^3$ is hydrogen.

In an embodiment, X is $-N(R^4)CH_2-$. In another embodiment, $R^4$ is methyl. In a preferred embodiment, X is $-N(R^4)CH_2-$ and $R^4$ is methyl. In a particularly preferred embodiment, $R^1$ is hydrogen, $R^2$ is methyl, $R^3$ is methyl, X is $-N(R^4)CH_2-$ and $R^4$ is methyl. In another particularly preferred embodiment, $R^1$ is methyl, $R^2$ is methyl, $R^3$ is hydrogen, X is $-N(R^4)CH_2-$ and $R^4$ is methyl.

In another particularly preferred embodiment, the compound of formula 1 has the structure of formula 1a:

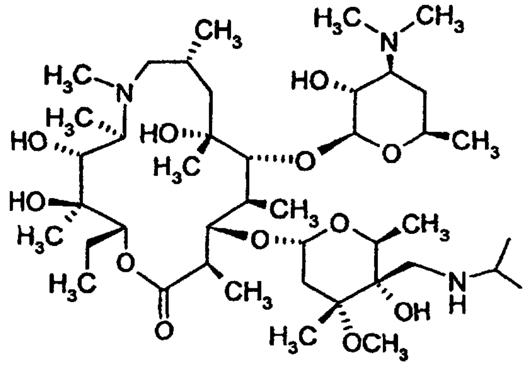

1a.

In another particularly preferred embodiment, the compound of formula 1 has the structure of formula 1b:

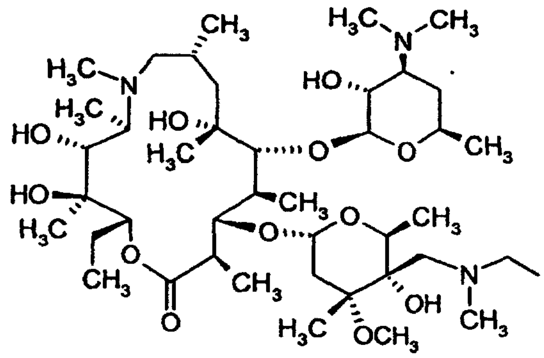

1b.

The term "compound(s) of formula 1" includes a compound of formula 1 as defined herein and all of the embodiments, preferred embodiments, more preferred embodiments, and particularly preferred embodiments thereof including the compounds of formulas 1a and 1b, which are particularly preferred embodiments of the compound. Accordingly, reference to a compound of formula 1 in connection with any of the embodiments, preferred embodiments, more preferred embodiments or particularly preferred embodiments of the compositions, processes and methods of the invention described herein is intended to refer to the compound of formula 1 as defined above, i.e., to any of its embodiments, preferred embodiments, more preferred embodiments or particularly preferred embodiments, especially the compounds of formulas 1a and 1b.

The compounds of formula 1 are 9a- azalides, according to the following numbering system used in, e.g., WO 98/56801 as shown in formula (I):

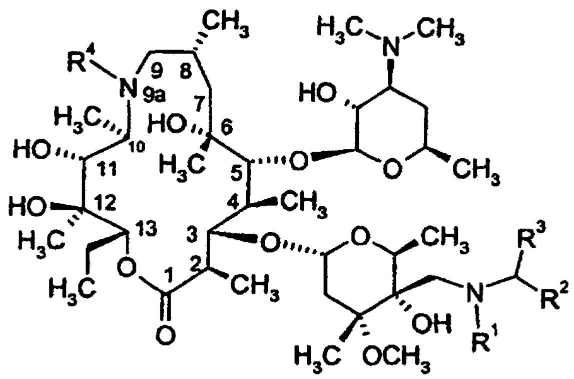

(I)

wherein when $R^4$ is methyl, the compound may be referred to as a "9a-N-methyl" azalide.

In an 8a-azalide, the compound of formula 1 has the structure shown in formula (II):

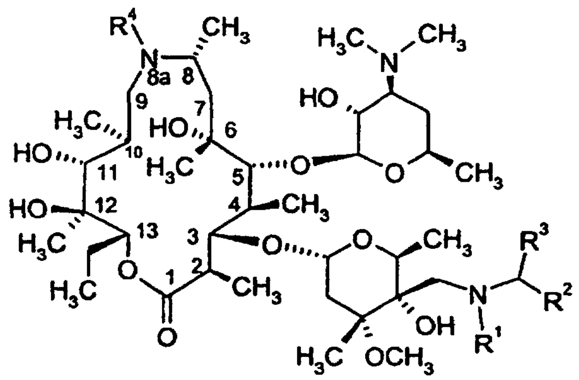

(II)

The term "azalide compound(sr as used in this application, unless otherwise specified, means a compound of formula 1 as that term is defined above. In an embodiment of the invention, the azalide compound is obtained from a preparation of substantially pure compound. "Substantially pure", as used herein, unless otherwise indicated, means having a purity of at least 97%.

In an embodiment of the invention, the one or more acids are selected from acetic acid, benzenesulfonic acid, citric acid, hydrobromic acid, hydrochloric acid, D- and L-lactic acid, methanesulfonic acid, phosphoric acid, succinic acid, sulfuric acid, D- and L-tartaric acid, p-toluenesulfonic acid, adipic acid, aspartic acid, camphorsulfonic acid, 1,2-ethanedisulfonic acid, laurylsulfuric acid, glucoheptonic acid, gluconic acid, 3-hydroxy-2-naphthoic acid, 1-hydroxy-2-naphthoic acid, 2-hydroxyethanesulfonic acid, malic acid, mucic acid, nitric acid, naphthalenesulfonic acid, palmitic acid, D-glucaric acid, stearic acid, maleic acid, malonic acid, fumaric acid, benzoic acid, cholic acid, ethanesulfonic acid, glucuronic acid, glutamic acid, hippuric acid, lactobionic acid, lysinic acid, mandelic acid, napadisylic acid, nicotinic acid, polygalacturonic acid, salicylic acid, sulfosalicylic acid, tannic acid and tryptophanic acid. It is to be understood that the one or more acids may comprise a mixture(s) of two or more acids selected from the above group. In an embodiment of the invention, the one or more acids comprise at least citric acid. In another embodiment of the invention, the amount of the compound of formula 1 is from 0.01 mmol to 0.3 mmol per mL of composition. In another embodiment of the invention, the one or more acids comprise at least citric acid, and the amount of citric acid is substantially the same as the amount of the compound of formula 1.

In another embodiment of the invention, the one or more acids are citric acid and hydrochloric acid, wherein the hydrochloric acid is present in an amount sufficient to achieve a pH of the composition of from 4.5 to 6.5.

In an embodiment of the invention, propylene glycol is present in an amount of from 40% to 75% by weight relative to the total volume. In a preferred embodiment, propylene glycol is present in an amount of from 40% to 75% by weight relative to the total volume and the pH of the composition is from 4.5 to 6.0. In a more preferred embodiment of the invention, the pH of the composition and the amount of propylene glycol are selected from the values within regions D, E and F shown in Figure 1. In another more preferred embodiment of the invention, the pH of the composition and the amount of propylene glycol are selected from the values within regions D, E and F shown in Figure 1, and $R^1$

is hydrogen, $R^2$ is methyl, $R^3$ is methyl, X is $-N(R^4)CH_2-$ and $R^4$ is methyl.

In an embodiment of the invention, propylene glycol is present in an amount of from 57% to 75% by weight relative to the total volume. In a preferred embodiment of the invention, propylene glycol is present in an amount of from 57% to 75% by weight relative to the total volume and the pH of the composition is from 4.7 to 5.6. In a more preferred embodiment of the invention, the pH of the composition and the amount of propylene glycol are selected from the values within regions E and F shown in Figure 1.

In an embodiment of the invention, propylene glycol is present in an amount of from 70% to 75% by weight relative to the total volume. In a preferred embodiment of the invention, propylene glycol is present in an amount of from 70% to 75% by weight relative to the total volume and the pH of the composition is from 4.8 to 5.2. In a more preferred embodiment of the invention, the pH of the composition and the amount of propylene glycol are selected from the values within region F shown in Figure 1.

In an embodiment of the invention, the pH of the composition is from 4.5 to 6.1. In a preferred embodiment of the invention, the pH of the composition and the amount of propylene glycol are selected from the values within regions D and E shown in Figure 2. In another preferred embodiment of the invention, the pH of the composition and the amount of propylene glycol are selected from the values within regions D and E shown in Figure 2; and $R^1$ is methyl, $R^2$ is methyl, $R^3$ is hydrogen, X is $-N(R^4)CH_2-$ and $R^4$ is methyl.

In an embodiment of the invention, propylene glycol is present in an amount of from 60% to 75% by weight relative to the total volume. In a preferred embodiment of the invention, the pH of the composition is from 4.8 to 5.5. In a more preferred embodiment of the invention, the pH of the composition and the amount of propylene glycol are selected from the values within region E shown in Figure 2.

In an embodiment of the invention, the composition further comprises one or more antioxidants present in an amount of from 0.01 mg to 10 mg per mL of the composition. In a preferred embodiment of the invention, the one or more antioxidants are selected from the group consisting of sodium bisulfite, sodium sulfite, sodium metabisulfite, sodium thiosulfate, sodium formaldehyde sulfoxylate, L-ascorbic acid, erythorbic acid, acetylcysteine, cysteine, monothioglycerol, thioglycollic acid, thiolactic acid, thiourea, dithiothreitol, dithioerythreitol, glutathione, ascorbyl palmitate, butylated hydroxyanisole, butylated hydroxytoluene, nordihydroguaiaretic acid, propyl gallate, $\alpha$-tocopherol, and mixtures thereof. In a more preferred embodiment of the invention, the one or more antioxidants is monothioglycerol, and in a particularly preferred embodiment thereof, the monothioglycerol is present in an amount of from 1 mg to 8 mg per mL of the composition.

In an embodiment of the invention, the composition further comprises one or more preservatives present in an amount of from 0.01 mg to 10 mg per mL of the composition. In a preferred embodiment, the one or more preservatives are selected from the group consisting of benzalkonium chloride, benzethonium chloride, benzoic acid, benzyl alcohol, methylparaben, ethylparaben, propylparaben, butylparaben, sodium benzoate, phenol, and mixtures thereof. In a more preferred embodiment of the invention, the one or more preservatives are selected from the group consisting of benzyl alcohol, methylparaben, propylparaben, a methylparaben/propylparaben combination, and phenol. In a particularly preferred embodiment of the invention, the one or more preservatives is phenol present in an amount of from 2 mg to 5 mg per mL of the composition.

The present invention also relates to a method for preparing an azalide composition as described herein, comprising the steps of:

(a) adding to water one or more acids, wherein the total amount of the one or more acids is sufficient to produce a solution having an acid concentration of from 0.01 to 0.3 mmol per mL of the composition;

(b) adding to the solution of step (a) propylene glycol in an amount sufficient to produce a concentration of propylene glycol of from 25% to 75% by weight relative to the volume of the composition and a compound of formula 1 in an amount sufficient to produce a concentration of the compound of from 0.01 to 0.3 mmol per mL of the composition; and

(c) adjusting the pH of the solution of step (c) to provide a pH of the composition of from 4.5 to 6.5.

In an embodiment of the invention, the method is carried out at ambient temperature, with consideration given to local manufacturing conditions, but preferably a temperature in the range from 20°C to 25°C. In an embodiment of the invention, the method further comprises stabilizing the solution by adding one or more antioxidants in an amount of from 0.01 mg to 10 mg per mL of the composition. In a preferred embodiment of the invention, the one or more antioxidants are selected from the group consisting of sodium bisulfite, sodium sulfite, sodium metabisulfite, sodium thiosulfate, sodium formaldehyde sulfoxylate, L-ascorbic acid, erythorbic acid, acetylcysteine, cysteine, monothioglycerol ("MTG"), thioglycollic acid, thiolactic acid, thiourea, dithiothreitol, dithioerythreitol, glutathione, ascorbyl palmitate, butylated hydroxyanisole, butylated hydroxytoluene, nordihydroguaiaretic acid, propyl gallate, $\alpha$-tocopherol, and mixtures thereof. In a more preferred embodiment of the the of the invention, the antioxidant is monothioglycerol present in an amount of from 1 mg to 8 mg per mL of composition.

In another embodiment of the invention, the method further comprises adding one or more preservatives in an amount of from about to 0.01 mg to 10 mg per mL of the composition. In a preferred embodiment of the invention, the one or more preservatives are selected from benzalkonium chloride, benzethonium chloride, benzoic acid, benzyl alcohol, methylparaben, ethylparaben, propylparaben, butylparaben, sodium benzoate, phenol, and mixtures thereof. In a more preferred embodiment of the invention, the preservative is phenol in an amount of from 2 mg to 5 mg per mL of the composition.

In an embodiment of the invention, the method further comprises after step (c), sterilizing the composition. In a preferred embodiment, the composition is sterilized by filtration.

This invention also relates to a method for treating a bacterial or protozoal infection in a mammal, comprising administering to a mammal in need of such treatment a therapeutically effective amount of an azalide composition. In an embodiment of the invention, the bacterial or protozoal infection is selected from the group consisting of coccidiosis; swine respiratory disease; bovine respiratory disease; dairy cow mastitis; canine skin, soft tissue and urinary tract infections; and feline skin, soft tissue and urinary tract infections.

This invention also relates to a pharmaceutical composition for the treatment of a bacterial or protozoal infection in a mammal, comprising at least an azalide composition of the invention and optionally, a pharmaceutically acceptable carrier.

This invention also relates to a pharmaceutical composition comprising the composition of claim 1 and a pharmaceutically acceptable carrier, as is described in further detail hereinbelow. In an embodiment of the invention, the pharmaceutically acceptable carrier comprises a diluent.

## Brief Description of Drawings

Figure 1 shows a contour plot for the compound of formula 1a. The plot was prepared from the 12 week, 50°C compound potency data of the various azalide compositions provided in Table 1, using SAS-JMP® Version 3.2 Statistical Discovery software, which fits the contour plot using triangulation and interpolation. Region A represents the pH and propylene glycol ("PG") percentage exhibiting a potency of less than 80.0%; region B, from 80.0% to less than 85.0%; region C, from 85.0% to less than 90.0%; region D, from 90.0% to less than 95.0%; region E, from 95.0% to less than 100.0%; and region F, greater than or equal to 100.0%.

Figure 2 shows a contour plot for the compound of formula 1b, prepared from the data provided in Table 1 reporting the potency of the compound after 12 weeks at 50°C in various azalide compositions, using the same method as in Figure 1. Region A represents the pH and PG percentage exhibiting a potency of less than 80.0%; region B, from 80.0% to less than 85.0%; region C, from 85.0% to less than 90.0%; region D, from 90.0% to less than 95.0%; and region E, from 95.0% to less than 100.0%.

Figure 1a shows Figure 1 with gridlines, and Equation A, which is a calculation of potency retention for the compound of formula 1a, with given percentage propylene glycol ("PG") and pH values, based on the results from Table 1.

Equation A:

$$z\ (\%\ potency) = 124.15 + (0.188 \times \%PG) - (7.633 \times pH) - (0.212 \times (\%PG - 50) \times (pH - 5.5)) - (7.967 \times (pH - 5.5)^2) + (0.003 \times (\%PG - 50)^2)$$

Figure 2a shows Figure 2 with gridlines, and Equation B, which is a calculation of potency retention for the compound of formula 1b, with given percentage propylene glycol ("PG") and pH values, based on the results from Table 1.

Equation B:

$$z\ (\%\ potency) = 126.817 + (0.162 \times \%PG) - (7.367 \times pH) + (0.176 \times (\%PG-50) \times (pH-5.5)) - (10.100 \times (pH-5.5)^2) - (0.001 \times (\%PG-50)^2)$$

Detailed Description of the Invention

The present invention relates to aqueous compositions comprising (a) a compound of formula 1 as defined above, (b) propylene glycol in an amount of from about 25% to about 75%; and (c) one or more acids to provide a pH of the composition of from about 4.5 to about 6.5. Of the embodiments of the compositions, processes and methods described above, particularly preferred embodiments of the compound of formula 1 useful therein are the compounds of formulas 1a and 1b. The chemical name of the compound of formula 1a is 1-Oxa-6-azacyclopentadecan-15-one, 13-[[2,6-dideoxy-3-C-methyl-3-O-methyl-4-C-[[(1-methylethyl)amino]methyl]-α-L-ribo-hexopyranosyl]oxy]-2-ethyl-3,4,10-trihydroxy-3,5,6,8,10,12,14-heptamethyl-11-[[3,4,6-trideoxy-3-(dimethylamino)-β-D-xylohexopyranosyl]oxy]-, (2R,3S,4R,5R,8R,90R,11R,12S,13S,14R)-. The chemical name of the compound of formula 1b is 1-Oxa-6-azacyclopentadecan-15-one, 13-[[2,6-dideoxy-4-C-[(ethylmethylamino)methyl]-3-C-methyl-3-O-methyl-α-L-ribo-hexopyranosyl]oxy]-2-ethyl-3,4,10-trihydroxy-3,5,6,8,10,12,14-heptamethyl-11-[[3,4,6-trideoxy-3-(dimethylamino)-β-D-xylo-hexopyranosyl]oxy]-, (2R,3S,4R,5R,8R,10R,11R,12S,13S,14R)-. Methods for obtaining a compound of formula 1 are disclosed in International Publication Nos. WO 98/56801 and EP 508699 A1. The azalide compounds of the compositions of the present invention are active antibiotic agents. The compositions of this invention are useful in the preparation of parenteral formulations of azalide compounds, e.g., formulations for intravenous injection.

Without being bound by any theory, the invention is based in part on the discovery that an aqueous composition comprising an azalide compound and propylene glycol, wherein the pH of the composition is buffered by one or more acids, exhibits a high degree of stability, as measured by the retention of potency, compared to compositions lacking propylene glycol. The compositions of this invention can be consistently produced, processed and stored for extended periods of time. Thus, the compositions of the present invention are highly desirable.

As used in this application, the term "potency" means the amount of an azalide compound present in a "test" sample subjected to a particular set of experimental conditions during a particular time interval, relative to the amount of the azalide compound present in an otherwise identical "control" sample stored at 5°C for the same time interval. Upon completion of the experiment for a given sample, the test and control samples are typically subjected to appropriate processing steps to allow accurate determination of the amount of the azalide compound. Relative amounts of azalide compound in the test and control samples may be determined by any number of means which are well-known in the art, such as high performance liquid chromatography ("HPLC"), nuclear magnetic resonance spectroscopy ("NMR"), gas chromatography ("GC"), mass spectrometry ("MS"), liquid chromatography/mass spectrometry ("LC/MS"), GC/MS, and thin layer chromatography ("TLC").

The concentration of the azalide compound in the composition can vary from 0.01 mmol to 0.30 mmol per mL of composition, more preferably from 0.05 mmol to 0.25 mmol per mL of composition, and most preferably from 0.10 mmol to 0.15 mmol per mL of composition. The amount of azalide compound in the compositions ranges from 8 mg of azalide compound per mL of composition to 250 mg of azalide compound per mL of composition. Preferably, the composition comprises from 40 mg to 200 mg, more preferably, from 80 to 120 mg of azalide compound per mL of composition.

Acids which are suitable for practicing the present invention include, but are not limited to, inorganic acids, such as hydrochloric, hydrobromic, phosphoric, sulfuric and nitric acids; and organic acids such as acetic acid, benzenesulfonic acid, citric acid, D- and L-lactic acid, methanesulfonic acid, succinic acid, D- and L-tartaric acid, p-toluenesulfonic acid, adipic acid, aspartic acid, camphorsulfonic acid, 1,2-ethanedisulfonic acid, laurylsulfuric acid, glucoheptonic acid, gluconic acid, 3-hydroxy-2-naphthoic acid, 1-hydroxy-2-naphthoic acid, 2-hydroxyethanesulfonic acid, malic acid, mucic acid, naphthalenesulfonic acid, palmitic acid, D-glucaric acid, stearic acid, maleic acid, malonic acid, fumaric acid, benzoic acid, cholic acid, ethanesulfonic acid, glucuronic acid, glutamic acid, hippuric acid, lactobionic acid, lysinic acid, mandelic acid, napadisylic acid, nicotinic acid, polygalacturonic acid, salicylic acid, sulfosalicylic acid, tannic acid and tryptophanic acid, as well as mixtures thereof.

The amount of acid to be used is an amount which is determined to be sufficient to buffer the pH of the composition adequately for an extended period of time at or around the chosen pH, and may vary according to the acid or acids selected. Preferably, the one or more acids are citric and hydrochloric acid, more preferably citric acid. When present, citric acid is present preferably at a concentration of from 0.01 mmol to 0.3 mmol per mL of solution. Citric acid when present may be used advantageously at a concentration substantially the same as the concentration of the azalide compound. However, the precise amount of acid (the absolute amount or amount relative to the amount of the compound of formula 1) is not critical, so long as the pH of the resulting solution is adequately buffered over a prolonged period of time, i.e., maintained within the range specified in a particular embodiment of the invention, according to the degree of stability desired. For example, with other conditions being similar, the amount of an acid required to maintain the pH within the pH range of region F of Figure 1 (4.8 to 5.2) is typically, but not necessarily, higher than the amount required to maintain the pH within the pH range encompassed by regions D, E and F of Figure 1 (4.5 to 6.0). Those of skill in the art will recognize that the amount of acid required for a desired pH will vary also according to which acid is used, e.g., whether the acid is monobasic, dibasic or tribasic, and that, in order to maintain

a pH within the desired range, additional acid and/or a base may be added to the solution of acid and the azalide compound. Suitable bases include, but are not limited to, alkali metal hydroxides and carbonates, alkali metal bicarbonates, and alkaline earth hydroxides and carbonates. Sodium hydroxide and potassium hydroxide are preferred. The acids and bases described above are conveniently used in the form of their aqueous solutions.

The compositions of this invention are useful for treating a bacterial or protozoal infection in a mammal. The compositions of this invention are also useful as intermediates for the formation of other stabilized compositions.

Without being bound by any theory, applicants believe that a composition containing propylene glycol improves the stability of the azalide compounds, particularly when the pH is maintained in the ranges disclosed herein. The presence of propylene glycol may also mitigate any pain experienced upon injection of the compositions and pharmaceutical compositions of the invention..

The compositions of this invention can still further comprise one or more antioxidants. Antioxidants retard the rate of or prevent oxidative breakdown of the compositions. Suitable antioxidants include, but are not limited to, sodium bisulfite, sodium sulfite, sodium metabisulfite, sodium thiosulfate, sodium formaldehyde sulfoxylate, L-ascorbic acid, erythorbic acid, acetylcysteine, cysteine, monothioglycerol ("MTG"), thioglycollic acid, thiolactic acid, thiourea, dithiothreitol, dithioerythreitol, glutathione, ascorbyl palmitate, butylated hydroxyanisole, butylated hydroxytoluene, nordihydroguaiaretic acid, propyl gallate, $\alpha$-tocopherol, and mixtures thereof. Those of skill in the art will recognize that the amount of antioxidant will vary according to which antioxidant is used. In a preferred embodiment, the antioxidant, when present, is present in an amount of from 0.01 mg to 10 mg per mL of composition. In a more preferred embodiment, the antioxidant is monothioglycerol. In a particularly preferred embodiment, the antioxidant is monothioglycerol present in an amount of from 1 mg to 8 mg per mL of composition. In another particularly preferred embodiment, the antioxidant is monothioglycerol present in an amount of from 4 mg to 6 mg per mL of composition.

The compositions of this invention optionally comprise one or more preservatives. Preservatives are useful for retarding the rate of or preventing proliferation of microorganisms, particularly when the compositions are exposed to air. Useful preservatives are: effective against a broad spectrum of microorganisms; physically, chemically and microbiologically stable over the lifetime of the compositions; non-toxic; adequately soluble; compatible with other components of the composition; and acceptable with respect to taste and odor. Suitable preservatives include, but are not limited to, benzalkonium chloride, benzethonium chloride, benzoic acid, benzyl alcohol, methylparaben, ethylparaben, propylparaben, butylparaben, sodium benzoate, phenol, and mixtures thereof. In a preferred embodiment, the one or more preservatives are selected from the group consisting of benzyl alcohol, methylparaben, propylparaben, a methylparaben/propylparaben combination, and phenol. When present, the one or more preservatives are present in an amount of from 0.01 to 10 mg per mL of the compositions. Preferably, the one or more preservatives is phenol. More preferably, the preservative is phenol present in an amount of from 2.0 to 5.0 mg per mL, more preferably, from 2.0 to 3.0 mg per mL, of the compositions. One of skill in the art will recognize that the amount of preservative to be used in the present compositions will depend on which preservative is chosen.

The compositions of this invention can be prepared as follows:

Reagents are added in a stainless steel- or glass-lined jacketed vessel, preferably with optional nitrogen overlay to reduce oxygen exposure of the mixtures in the compounding vessel during manufacture.

A portion of the final amount of Water for Injection ("WFI") is added to the reaction vessel, and agitation is begun. Each additional component is added while the mixture is continuously agitated. Preferably, the first step is addition of an acid, in an amount which will yield a final concentration in the composition of 0.01 mmol to 0.3 mmol per mL of composition, which preferably is allowed to dissolve before the next component is added. In a preferred embodiment, the acid is citric acid, more preferably, anhydrous citric acid (preferably USP grade). Next, either the compound of formula 1 or the propylene glycol is added; the order of addition is not critical, i.e., either one may be added before the other. The compound of formula 1 is added in an amount sufficient to yield a final concentration in the composition of from 0.01 to 0.3 mmol per mL of composition. The propylene glycol is added in an amount which will yield the desired final concentration of from 25% to 75% by weight relative to the total volume. After the addition of either compound, the mixture is allowed to agitate until the added ingredient has dissolved. Preferably, the propylene glycol is added after the acid and before the addition of the compound of formula 1. Preferably, after addition of at least the compound of formula 1, the pH of the mixture is adjusted to a value in a range as set forth in the various embodiments, preferred embodiments and particularly preferred embodiments of the compositions of the invention. The adjustment of pH is preferably accomplished using a concentrated aqueous solution of hydrochloric acid to decrease the pH and a concentrated aqueous solution of sodium hydroxide to increase the pH, accompanied by agitation. It is anticipated that regular practice of the invention by the skilled practitioner will allow the predetermination of the amount of acid and/or base needed for adjustment of pH, allowing the adjustment to be carried out preferably as a single addition step, preferably performed at any point after addition of the one or more acids used to buffer the pH. Finally, the mixture is diluted, using water for injection, to achieve the final concentration of the components in the composition. For use as a pharmaceutical composition, the composition is also sterilized, preferably by filtration.

Before dilution to the final volume, antioxidant is optionally added in an amount of from 0.01 mg to 10 mg per

mL of the composition. If present, preservative is added also before dilution to the final volume, in an amount of from about 0.01 to about 10 mg per mL of the composition, and the pH is re-adjusted to the desired pH by adding acid and/ or base, for example, as a 10% (w/w) aqueous solution or in solid form. The resulting composition is diluted to a desired volume. In one embodiment, the final concentration of the azalide compound is 0.01 mmol to 0.30 mmol, preferably 0.05 mmol to 0.25 mmol, and most preferably 0.10 mmol to 0.15 mmol per mL of the resulting composition.

The resulting compositions are preferably sterilized, for example, by passing the compositions through a pre-filter, *e.g.*, a 5-10 micron filter and then through a 0.2 micron final sterilizing filter that has been previously sterilized. The sterilizing filter is sterilized by moist-heat autoclaving for 60 minutes at 121°C, and is tested for integrity using a pressure-hold method prior to sterilization and after product filtration.

The sterile composition is added to suitable containers, preferably, *e.g.*, glass vials. For example, the compositions may be stored in 20 mL flint type I serum glass vials (Wheaton Science Products, Millville, New Jersey) which are sterilized and depyrogenated in a dry heat tunnel at 250°C for 240 minutes. 20 mm 4432/50 gray chlorobutyl siliconized stoppers (The West Company, Lionville, PA) are depyrogenated by washing and sterilized by moist-heat autoclaving for 60 minutes at 121°C. For pharmaceutical use, each of the vials is filled under sterile conditions, the vial head spaces are flushed with nitrogen, and the vials are sealed with rubber stoppers and an appropriate overseal, e.g., an aluminum crimp. Those skilled in the art will recognize that modifications to the above can be used to prepare sterile compositions.

Preferred methods for determining that a stable azalide composition has been obtained include gel chromatography, thin-layer chromatography, and HPLC. More preferably, HPLC is used. To monitor the compositions of the invention for potency, after dilution to final volume and sterilization by filtration, the compositions were subdivided into 3.5 mL glass vials which were sealed with rubber stoppers and an aluminum crimp. For the tests reported below, approximately 10 vials containing 2.5 mL each were prepared.

The present invention also relates to a pharmaceutical composition. A pharmaceutical composition of this invention comprises at least an azalide composition as that term is defined herein (which includes each of the embodiments, preferred, more preferred and particularly preferred embodiments of the azalide compositions described herein), and optionally contains one or more inactive ingredients (i.e., other than the azalide compound) in addition to propylene glycol and one or more acids, which components are encompassed by the term "pharmaceutically acceptable carrier." In other words, an azalide composition of this invention is one embodiment of a pharmaceutical composition, wherein the propylene glycol and one or more acids together comprise a pharmaceutically acceptable carrier, and in other embodiments, the pharmaceutical composition further contains additional ingredients, such as excipients, diluents, etc. which are pharmaceutically acceptable carriers and are present in the composition in order to provide a variety of additional pharmaceutical compositions of the invention.

In another embodiment, the pharmaceutical composition comprises an azalide composition and a diluent. Examples of diluents include, but are not limited to, sterile saline, electrolyte replenishment solutions, and dextrose solutions. Depending on the amount of the buffered aqueous propylene glycol composition, and its initial pH, the pH of the final pharmaceutical composition may differ from the pH of the azalide composition, and the pH of the pharmaceutical composition may be outside the pH range (4.5 to 6.5) of the azalide composition. The invention also relates to a pharmaceutical composition prepared by combining an azalide composition of the invention and a pharmaceutically acceptable carrier. In preferred embodiments of the inventions relating to methods of treating diseases as described herein, the pharmaceutical composition is administered to the mammal in need thereof before the potency of the composition decreases below the range set forth herein for such composition. For example, a composition of the invention having the amount of propylene glycol and pH encompassed by regions D, E and F of Figure 1, a pharmaceutical composition prepared from such azalide composition is administered before the potency decreases below 90%. An example of a pharmaceutical composition whose pH may lie outside the range of the pH of the azalide composition, is in the use of the composition in combination with an electrolyte replenishment solution, which is typically buffered to a pH of 7.4; depending on the concentration of acid and azalide compound in the final pharmaceutical composition, the pH of the pharmaceutical composition may be higher than 6.5, e.g., up to 7.4.

In an embodiment, the pharmaceutical composition comprises an azalide composition and a pharmaceutically acceptable carrier. Some examples of suitable pharmaceutically acceptable carriers include (in addition to propylene glycol and one or more acids already present in the azalide composition), inert diluents or fillers, various organic solvents, binders and

excipients, including ingredients useful for enhancing palatability, e.g., flavorings. Other pharmaceutically acceptable carriers useful in preparing compositions for oral administration, e.g., tablets, include disintegrants such as starch, alginic acid and certain complex silicates and with binding agents such as sucrose, gelatin and acacia. Additional pharmaceutically acceptable carriers include lubricating agents such as magnesium stearate, sodium lauryl sulfate and talc are often useful for tableting purposes. Solid compositions of a similar type may also be employed in soft and hard filled gelatin capsules; preferred materials therefor include the pharmaceutically acceptable carriers lactose, milk sugar and high molecular weight polyethylene glycols. When aqueous suspensions or elixirs are desired for oral administration the azalide composition may be combined with various sweetening or flavoring agents, coloring matters or dyes and, if desired, emulsifying agents or suspending agents, together with diluents such as water, ethanol, glycerin, or combinations thereof.

Methods of preparing various pharmaceutical compositions with a specific amount of active compound are known, or will be apparent, to those skilled in this art. For examples, see Remington: The Practice of Pharmacy, Lippincott Williams and Wilkins, Baltimore MD, 20th ed. 2000.

A pharmaceutical composition of the present invention optionally may further contain a second active ingredient, such as a biological component, e.g., an antigen, vitamins, minerals or dietary supplement. Other active ingredients may include immunomodulators such as interferons, interleukins and other cytokines, non-steroidal anti-inflammatory compounds such as propionic acid derivatives (e.g., ibuprofen, ketoprofen, naproxen, benoxprofen, carprofen), acetic acid derivatives (e.g., acemetacin, alclofenac, clidanac, diclofenac, fenclofenac, fenclozic acid, fentiazac, furofenac, ibufenac, isoxepac, oxpinac, sulindac, tiopinac, tolmetin, zidometacin, and zomepirac), fenamic acid derivatives (e.g., flufenamic acid, meclofenamic acid, mefenamic acid, niflumic acid and tolfenamic acid), biphenylcarboxylic acids (e.g., diflufenisal, flufenisal), and cyclooxygenase-2 (COX-2) inhibitors, antiparasitic agents such as avermectin, ivermectins, milbemycins, levamisole, benzimidazoles, imidazolidinones, pyrantel/morantel.

The present invention also relates to methods for treating a mammal, comprising administering to a mammal in need of such treatment a pharmaceutically effective amount of a composition of the invention. The compositions of the invention can be used to treat infections by gram-positive bacteria, gram-negative bacteria, protozoa, and mycoplasma, including, but not limited to, *Actinobacillus pleuropneumonia, Pasteurella multocida, Pasteurella haemolytica, H. parasuis, B. bronchiseptica, S. choleraesuis, S. pilo, Moraxella bovis, H. somnus, M. bovis, Eimeria zuernii, Eimeria bovis, A. marginale, M. hyopneumoniae, Lawsonia intracellularis*, and *staphylococcus, salmonella, chlamydia, coccidia, cryptosporidia, E. coli, haemophilus, neospora,* and *streptococcus* species.

The term "treatment", as used herein, unless otherwise indicated, includes the treatment or prevention of a bacterial infection or protozoal infection as provided in the method of the present invention.

As used herein, unless otherwise indicated, the terms "bacterial infection(s)" and "protozoal infection(s)" include bacterial infections and protozoal infections that occur in mammals, fish and birds as well as disorders related to bacterial infections and protozoal infections that may be treated or prevented by administering antibiotics such as the compounds of the present invention. Such bacterial infections and protozoal infections, and disorders related to such infections, include the following: pneumonia, otitis media, sinusitus, bronchitis, tonsillitis, and mastoiditis related to infection by *Streptococcus pneumoniae*, *Haemophilus influenzae, Moraxella catarrhalis, Staphylococcus aureus*, or *Peptostreptococcus* spp.; pharyngitis, rheumatic fever, and glomerulonephritis related to infection by *Streptococcus pyogenes*, Groups C and G streptococci, *Clostridium diptheriae*, or *Actinobacillus haemolyticum;* respiratory tract infections related to infection by *Mycoplasma pneumoniae, Legionella pneumophila, Streptococcus pneumoniae, Haemophilus influenzae*, or *Chlamydia pneumoniae;* uncomplicated skin and soft tissue infections, abscesses and osteomyelitis, and puerperal fever related to infection by *Staphylococcus aureus*, coagulase-positive staphylococci (i.e., *S. epidermidis, S. hemolyticus*, etc.), *Streptococcus pyogenes , Streptococcus agalactiae*, Streptococcal groups C-F (minute-colony streptococci), viridans streptococci, *Corynebacterium minutissimum, Clostridium* spp., or *Bartonella henselae;* uncomplicated acute urinary tract infections related to infection by *Staphylococcus saprophyticus* or *Enterococcus* spp.; urethritis and cervicitis; and sexually transmitted diseases related to infection by *Chlamydia trachomatis, Haemophilus ducreyi, Treponema pallidum, Ureaplasma urealyticum*, or *Neiserria gonorrheae;* toxin diseases related to infection by *S. aureus* (food poisoning and Toxic shock syndrome), or Groups A, B, and C streptococci; ulcers related to infection by *Helicobacter pylori;* systemic febrile syndromes related to infection by *Borrelia recurrentis;* Lyme disease related to infection by *Borrelia burgdorferi;* conjunctivitis, keratitis, and dacrocystitis related to infection by *Chlamydia trachomatis, Neisseria gonorrhoeae, S. aureus, S. pneumoniae, S. pyogenes, H. influenzae*, or *Listeria* spp.; disseminated *Mycobacterium avium* complex (MAC) disease related to infection by *Mycobacterium avium*, or *Mycobacterium intracellulare;* gastroenteritis related to infection by *Campylobacter jejuni;* intestinal protozoa related to infection by *Cryptosporidium* spp.; odontogenic infection related to infection by viridans streptococci; persistent cough related to infection by *Bordetella pertussis;* gas gangrene related to infection by *Clostridium perfringens* or *Bacteroides* spp.; and atherosclerosis related to infection by *Helicobacter pylori* or *Chlamydia pneumoniae*. Bacterial infections and protozoal infections and disorders related to such infections that may be treated or prevented in animals include the following: bovine respiratory disease related to infection by *P. haem., P. multocida, H. Somnus, Mycoplasma* spp.; cow enteric disease related to infection by *E. coli* or protozoa (i.e., coccidia, cryptosporidia, etc.); dairy cow mastitis related to infection by *Staph. aureus, Strep. uberis, Strep. agalactiae, Strep. dysgalactiae, Klebsiella* spp., *Corynebacterium*, or *Enterococcus* spp.; swine respiratory disease related to infection by *A. pleuro., P. multocida*, or *Mycoplasma* spp.; swine enteric disease related to infection by *E. coli, Lawsonia intracellularis, Salmonella,* or *Serpulina hyodyisinteriae*; cow footrot related to infection by *Fusobacterium* spp.; cow metritis related to infection by *E. coli;* cow hairy warts related to infection by *Fusobacterium necrophorum* or *Bacteroides nodosus;* cow pink-eye related to infection by *Moraxella bovis;* cow premature abortion related to infection by protozoa (i.e. neosporium); urinary tract infection in dogs and

cats related to infection by *E. coli;* skin and soft tissue infections in dogs and cats related to infection by *Staph. epidermidis*, *Staph. intermedius, coagulase neg. Staph.* or *P. multocida;* and dental or mouth infections in dogs and cats related to infection by *Alcaligenes* spp., *Bacteroides* spp., *Clostridium* spp., *Enterobacter* spp., *Eubacterium, Peptostreptococcus, Porphyromonas*, or *Prevotella*. Other bacterial infections and protozoal infections and disorders related to such infections that may be treated or prevented in accord with the method of the present invention are referred to in J. P. Sanford et al., "The Sanford Guide To Antimicrobial Therapy," 26th Edition, (Antimicrobial Therapy, Inc., 1996). The compositions of the invention are particularly useful in treating infections such as coccidiosis; swine respiratory disease; bovine respiratory disease; dairy cow mastitis; canine skin, soft tissue and urinary tract infections; and feline skin, soft tissue and urinary tract infections.

The antibacterial and antiprotozoal activity of the compounds of the present invention against bacterial and protozoa pathogens is demonstrated by the compounds' ability to inhibit growth of defined strains of human or animal pathogens.

Assay I

This assay employs conventional methodology and interpretation criteria and is designed to test for activity against *Pasteurella multocida*, using the liquid dilution method in microliter format. A single colony of *P. multocida* (strain 59A067) is inoculated into 5 mL of brain heart infusion (BHI) broth. The azalide formulation is prepared by solubilizing 1 mg of the azalide composition in 125 μL of dimethylsulfoxide (DMSO). Dilutions of the azalide compositions are prepared using uninoculated BHI broth. The concentrations of the azalide compounds used range from 200 μg/mL to 0.098 μg/mL by two-fold serial dilutions. The *P. multocida* inoculated BHI is diluted with uninoculated BHI broth to make a $10^4$ cell suspension per 200 μL. The BHI cell suspensions are mixed with respective serial dilutions of the azalide compositions, and incubated at 37°C for 18 hours. The minimum inhibitory concentration (MIC) is equal to the concentration of the mixture exhibiting 100% inhibition of growth of P. multocida as determined by comparison with an uninoculated control.

In this assay, the compound of formula 1a exhibits a MIC against E. coli of 0.39 μg/mL; against P. multocida, 0.05 μg/mL, and against S. aureus, a MIC of 0.2 μg/mL. Also in this assay, the compound of formula 1b exhibits a MIC against E. coli of 0.39 μg/mL; against P. multocida, 0.05 μg/mL, and against S. aureus, a MIC of 0.39 μg/mL. Accordingly, as expected, the azalide compounds used in this invention are active against a variety of organisms.

Assay II

This assay is based on the agar dilution method using a Steers Replicator may be used to test for activity against *Pasteurella haemolytica*. Two to five colonies isolated from an agar plate are inoculated into BHI broth and incubated overnight at 37°C with shaking (200 rpm). The next morning, 300 μL of the fully grown *P. haemolytica* preculture is inoculated into 3 mL of fresh BHI broth and is incubated at 37°C with shaking (200 rpm). The appropriate amounts of the azalide compositions are dissolved in ethanol and a series of two-fold serial dilutions are prepared. Two mL of the respective serial dilution is mixed with 18 mL of molten BHI agar and solidified. When the inoculated *P. haemolytica* culture reaches 0.5 McFarland standard density, about 5 μL of the *P. haemolytica* culture is inoculated onto BHI agar plates containing the various concentrations of the azalide composition using a Steers Replicator and incubated for 18 hours at 37°C. Initial concentrations of the mixture range from 100-200 μg/mL. The MIC is equal to the concentration of the mixture exhibiting 100% inhibition of growth of *P. haemolytica* as determined by comparison with an uninoculated control.

Most preferably, microdilution assays are performed using cation-adjusted Mueller-Hinton broth according to NCCLS guideline M31-A, Vol. 19, No. 11, "Performance standards for antimicrobial disk and dilution susceptibility tests for bacteria isolated from animals," June 1999 (ISBN 1-56238-377-9), which is herein incorporated by reference. This assay may be used to determine the MIC of a compound against not only *P. haemolytica* and *P. multocida* but also a variety of other organisms.

Assay III

The in vivo activity of the compositions of the present invention can be determined by conventional animal protection studies well known to those skilled in the art, usually carried out in mice.

Mice are allotted to cages (10 per cage) upon their arrival, and allowed to acclimate for a minimum of 48 hours before being used. Animals are inoculated with 0.5 ml of a 3 x $10^3$ CFU/ml bacterial suspension (*P. multocida* strain 59A006) intraperitoneally. Each experiment has at least 3 non-medicated control groups including one infected with 0.1X challenge dose and two infected with 1X challenge dose; a 10X challenge data group may also be used. Generally, all mice in a given study can be challenged within 30-90 minutes, especially if a repeating syringe (such as a Cornwall

syringe) is used to administer the challenge. Thirty minutes after challenging has begun, the first pharmaceutical composition treatment is given. It may be necessary for a second person to begin pharmaceutical composition dosing if all of the animals have not been challenged at the end of 30 minutes. The routes of administration are subcutaneous or oral doses. Subcutaneous doses are administered into the loose skin in the back of the neck whereas oral doses are given by means of a feeding needle. In both cases, a volume of 0.2 ml is used per mouse. Compositions are administered 30 minutes, 4 hours, and 24 hours after challenge. A control composition of known efficacy administered by the same route is included in each test. Animals are observed daily, and the number of survivors in each group is recorded. The *P. multocida* model monitoring continues for 96 hours (four days) post challenge.

The $PD_{50}$ is a calculated dose at which the composition tested protects 50% of a group of mice from mortality due to the bacterial infection which would be lethal in the absence of treatment.

The compositions of the invention can be used to treat humans, cattle, horses, sheep, swine, goats, rabbits, cats, dogs, and other mammals in need of such treatment. In particular, the compositions of the invention can be used to treat, *inter alia*, bovine respiratory disease, swine respiratory disease, pneumonia, pasteurellosis, coccidiosis, anaplasmosis, and infectious keratinitis. The compositions may be administered through oral, intramuscular, intravenous, subcutaneous, intra-ocular, parenteral, topical, intravaginal, or rectal routes. For administration to cattle, swine or other domestic animals, the compositions may be administered in feed or orally as a drench composition. Preferably, the compositions are injected intramuscularly, intravenously or subcutaneously. In a preferred embodiment, the compositions are administered in dosages ranging from about 0.5 mg of the azalide compound per kg of body weight per day (mg/kg/day) to about 20 mg/kg/day. In a more preferred embodiment, the compositions are administered in dosages of the azalide compound ranging from about 1 mg/kg/day to about 10 mg/kg/day. In a most preferred embodiment, the compositions are administered in dosages of azalide compound ranging from about 1.25 mg/kg/day to about 5.0 mg/kg/day. The compositions can be administered up to several times per day, for about 1 to about 15 days, preferably about 1 to about 5 days, and repeated where appropriate. Those of skill in the art will readily recognize that variations in dosages can occur depending upon the species, weight and condition of the subject being treated, its individual response to the compositions, and the particular route of administration chosen. In some instances, dosage levels below the lower limit of the aforesaid ranges may be therapeutically effective, while in other cases still larger doses may be employed without causing any harmful side effects, provided that such larger doses are first divided into several small doses for administration throughout the day.

The following Examples further illustrate the compositions and methods of the present invention. It is to be understood that the present invention is not limited to the specific details of the Examples provided below.

Examples

Approximately 30 mL of each composition listed in Table 1 below was prepared as follows:

1) Sufficient citric acid was added to Water for Injection to produce a solution having a final concentration of acid of 0.1 M;
2) The compound of formula 1a or 1b was added in an amount to produce a final azalide concentration of 100 mg/mL (0.12 mmol/mL of the composition), and the mixture was stirred until the azalide was dissolved;
3) Propylene glycol was added to produce a concentration in the final mixture of from 25% to 75% by weight relative to the total volume;
4) The pH was adjusted by addition of 10% aqueous HCl or 10 M aqueous NaOH as necessary;
5) Water for Injection was added to bring the volume to 30 mL;
6) Each sample was subdivided by filtration through a 0.22 micron Millex GV filter into 3.5 mL glass vials which were each sealed with a rubber stopper and an overseal consisting of an aluminum crimp. Approximately 10 vials containing 2.5 mL each were prepared for each sample.

To monitor the stability of the azalide compositions, vials were assayed by HPLC after exposure to elevated temperatures for a period of time. Aliquots were removed from vials and diluted with a mixture of 15% 10 mM potassium phosphate buffer, pH 6.5, 85% 1:1 (v/v) acetonitrile:methanol to a concentration of approximately 1.0 mg of the compound of formula 1 per mL total sample volume. Diluted samples were subjected to chromatography in a Waters Alliance 2690 HPLC system having a 2487 UV detector. The column was a Waters Symmetry C-18, 5 micron (250x4.6 mm), the mobile phase was the same as the phosphate/acetonitrile/methanol diluent above and the flow rate was 1 mL/minute. Peaks were detected by monitoring ultraviolet absorption at 210 nm. Relative amounts of azalide compound in the experimental samples and the controls were determined by taking the ratio of their relative chromatogram-peak areas. Although HPLC was used in this experiment, other known methods, including NMR, GC, MS, LC/MS, GC/MS, and TLC can be used to evaluate the potency of the compound of formula 1.

The results are reported in Table 1 below as a percentage of the compound of formula 1 remaining after

exposure to various temperature and duration conditions, relative to the amount of the compound present in an otherwise identical sample stored at 5°C for the same time interval.

Table 1 -

| Potency retention data | | | | | |
|---|---|---|---|---|---|
| Compound | Preparation number | Propylene glycol concentration (w/v %) | PH | Potency (% of 5°C) | |
| | | | | 6 weeks, 50°C | 12 weeks, 50°C |
| 1a | 1 | 50 | 4.5 | 91.9 | 91.3 |
| 1a | 2 | 25 | 5.0 | 93.6 | 87.9 |
| 1a | 3 | 75 | 5.0 | 96.0 | 102.6 |
| 1a | 4 | 50 | 5.5 | 96.3 | 92.9 |
| 1a | 5 | 50 | 5.5 | 95.6 | 91.4 |
| 1a | 6 | 50 | 5.5 | 93.9 | 90.4 |
| 1a | 7 | 25 | 6.0 | 90.6 | 85.7 |
| 1a | 8 | 75 | 6.0 | 89.5 | 89.8 |
| 1a | 9 | 50 | 6.5 | 84.1 | 75.9 |
| | | | | | |
| 1b | 1 | 50 | 4.5 | 96.1 | 92.1 |
| 1b | 2 | 25 | 5.0 | 96.5 | 92.4 |
| 1b | 3 | 75 | 5.0 | 98.8 | 96.1 |
| 1b | 4 | 50 | 5.5 | 94.9 | 91.8 |
| 1b | 5 | 50 | 5.5 | 93.0 | 96.1 |
| 1b | 6 | 50 | 5.5 | 93.3 | 95.3 |
| 1b | 7 | 25 | 6.0 | 90.0 | 81.5 |
| 1b | 8 | 75 | 6.0 | 93.8 | 94.0 |
| 1b | 9 | 50 | 6.5 | 86.5 | 76.5 |

The data provided in Table 1 at 50°C, 12 weeks were used to construct Figure 1 and Figure 2, for compounds 1a and 1b, respectively, using SAS-JMP Version 3.2 Statistical Discovery software, by triangulation and interpolation to construct the contours between the data points. The contours represent lines of equal potency. The results of these experiments indicate that after storage for 12 weeks at 50'C, higher potency is achieved by increasing the amount of propylene glycol, preferably to 40% or greater, or more preferably to 60% or greater, and even more preferably to 70 - 75%, in an aqueous solution having a generally acidic pH. The range of simultaneous pH conditions and propylene glycol concentrations which are most desirable yield a potency of 90% or better, preferably 95% or better after 12 weeks at 50°C, and are readily selectable using Figures 1 and 2. Notably, the results for the two compounds are most similar in the region of highest propylene glycol concentrations, i.e., between 60% and 75% propylene glycol and having a pH range providing the highest potency at those concentrations, i.e., generally less than 5.5 and above 4.7, which region is most accurately indicated in regions E and F of Figure 1 and region E in Figure 2.

## Claims

**1.** An aqueous composition comprising:

(a) a compound of formula 1

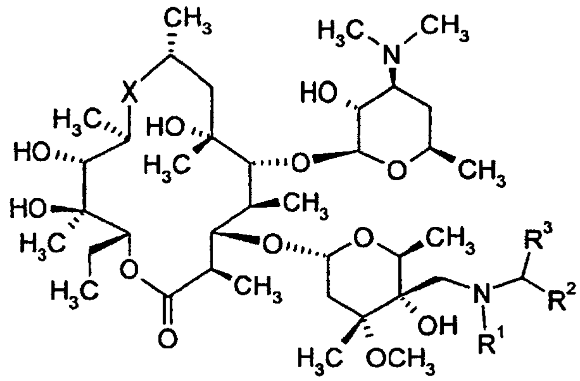

1 ;

(b) propylene glycol in an amount of from about 25% to about 75% by weight relative to the total volume;
(c) one or more acids to provide a pH of the composition of from 4.5 to 6.5; and
(d) water;

wherein

$R^1$ and $R^2$ are each independently selected from the group consisting of hydrogen, $(C_1\text{-}C_{10})$alkyl, and $(C_3\text{-}C_7)$ cycloalkyl;

$R^3$ is selected from the group consisting of hydrogen and $(C_1\text{-}C_{10})$alkyl;

X is $-N(R^4)CH_2-$ or $-CH_2N(R^4)-$; and

$R^4$ is $(C_1\text{-}C_3)$alkyl.

**2.** The composition of claim 1, wherein X is $-N(R^4)CH_2-$ and $R^4$ is methyl, and wherein $R^2$ is methyl.

**3.** The composition of claim 2, wherein either $R^1$ is methyl and $R^3$ is hydrogen, or $R^1$ is hydrogen and $R^3$ is methyl.

**4.** The composition of claims 1, 2 or 3, wherein the one or more acids are selected from the group consisting of acetic acid, benzenesulfonic acid, citric acid, hydrobromic acid, hydrochloric acid, D- and L-lactic acid, methanesulfonic acid, phosphoric acid, succinic acid, sulfuric acid, D- and L-tartaric acid, p-toluenesulfonic acid, adipic acid, aspartic acid, camphorsulfonic acid, 1,2-ethanedisulfonic acid, laurylsulfuric acid, glucoheptonic acid, gluconic acid, 3-hydroxy-2-naphthoic acid, 1-hydroxy-2-naphthoic acid, 2-hydroxyethanesulfonic acid, malic acid, mucic acid, nitric acid, naphthalenesulfonic acid, palmitic acid, D-glucaric acid, stearic acid, maleic acid, malonic acid, fumaric acid, benzoic acid, cholic acid, ethanesulfonic acid, glucuronic acid, glutamic acid, hippuric acid, lactobionic acid, lysinic acid, mandelic acid, napadisylic acid, nicotinic acid, polygalacturonic acid, salicylic acid, sulfosalicylic acid, tannic acid and tryptophanic acid.

**5.** The composition of claim 4, wherein the one or more acids comprise at least citric acid.

**6.** The composition of claim 5, wherein the amount of the compound of formula 1 is from 0.01 mmol to 0.3 mmol per mL of the composition.

**7.** The composition of claim 6, wherein the one or more acids are citric acid and hydrochloric acid, wherein the hydrochloric acid is present in an amount sufficient to achieve a pH of the composition of from 4.5 to 6.5.

**8.** The composition of claims 1, 2 or 3, wherein propylene glycol is present in an amount of from 40% to 75% by weight relative to the total volume.

**9.** The composition of claim 8, wherein the pH of the composition is from 4.5 to 6.0.

**10.** The composition of claim 9, wherein the pH of the composition and the amount of propylene glycol are selected

from the values within regions D, E and F shown in Figure 1.

**11.** The composition of claim 10, wherein propylene glycol is present in an amount of from 57% to 75% by weight relative to the total volume.

**12.** The composition of claim 11, wherein the pH of the composition is from 4.7 to 5.6.

**13.** The composition of claims 1, 2 or 3, which further comprises one or more antioxidants present in an amount of from 0.01 mg to 10 mg per mL of the composition.

**14.** The composition of claims 1, 2 or 3, which further comprises one or more preservatives present in an amount of from 0.01 mg to 10 mg per mL of the composition.

**15.** A method for preparing the composition of claims 1, 2 or 3, comprising the steps of:

(a) adding to water one or more acids, wherein the total amount of the one or more acids is sufficient to produce a solution having an acid concentration of from 0.01 to 0.3 mmol per mL of the composition;
(b) adding to the solution of step (a) propylene glycol in an amount sufficient to produce a concentration of propylene glycol of from 25% to 75% by weight relative to the volume of the composition and a compound of formula 1 in an amount sufficient to produce a concentration of the compound of from 0.01 to 0.3 mmol per mL of the composition; and
(c) adjusting the pH of the solution of step (c) to provide a pH of the composition of from 4.5 to 6.5.

## Patentansprüche

**1.** Wässrige Zusammensetzung, die umfasst:

(a) eine Verbindung der Formel 1

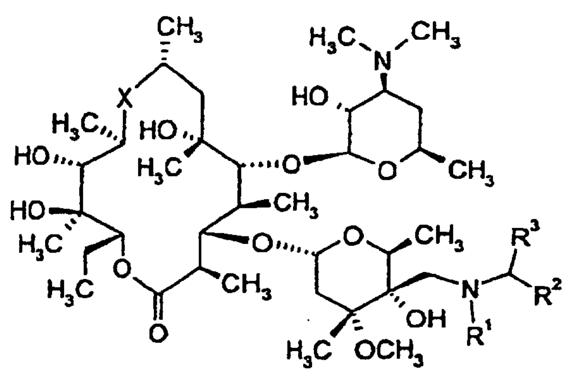

1;

(b) Propylenglykol in einer Menge von etwa 25 bis etwa 75 Gew.-%, bezogen auf das Gesamtvolumen,
(c) eine oder mehrere Säuren zur Bereitstellung eines pH-Werts der Zusammensetzung von 4,5 bis 6,5, und
(d) Wasser;

wobei
$R^1$ und $R^2$ jeweils unabhängig voneinander aus der Gruppe von Wasserstoff, $(C_1-C_{10})$Alkyl und $(C_3-C_7)$Cycloalkyl ausgewählt sind;
$R^3$ aus der Gruppe von Wasserstoff und $(C_1-C_{10})$Alkyl ausgewählt ist;
X $-N(R^4)CH_2-$ oder $-CH_2N(R^4)-$ bedeutet; und
$R^4$ $(C_1-C_3)$Alkyl bedeutet.

**2.** Zusammensetzung nach Anspruch 1, wobei X $-N(R^4)CH_2-$bedeutet und $R^4$ Methyl bedeutet, und wobei $R^2$ Methyl bedeutet.

3. Zusammensetzung nach Anspruch 2, wobei entweder $R^1$ Methyl und $R^3$ Wasserstoff bedeutet oder $R^1$ Wasserstoff und $R^3$ Methyl bedeutet.

4. Zusammensetzung nach Anspruch 1, 2 oder 3, wobei die eine oder mehreren Säuren ausgewählt sind aus der Gruppe von Essigsäure, Benzolsulfonsäure, Citronensäure, Bromwasserstoffsäure, Salzsäure, D- und L-Milchsäure, Methansulfonsäure, Phosphorsäure, Bernsteinsäure, Schwefelsäure, D- und L-Weinsäure, p-Toluolsulfonsäure, Adipinsäure, Asparaginsäure, Camphersulfonsäure, 1,2-Ethandisulfonsäure, Laurylschwefelsäure, Glucoheptonsäure, Gluconsäure, 3-Hydroxy-2-naphthoesäure, 1-Hydroxy-2-naphthoesäure, 2-Hydroxyethansulfonsäure, Äpfelsäure, Mucinsäure, Salpetersäure, Naphthalinsulfonsäure, Palmitinsäure, D-Glucarsäure, Stearinsäure, Maleinsäure, Malonsäure, Fumarsäure, Benzoesäure, Cholsäure, Ethansulfonsäure, Glucuronsäure, Glutaminsäure, Hippursäure, Lactobionsäure, Lysinsäure, Mandelsäure, Napadisylsäure, Nicotinsäure, Polygalacturonsäure, Salicylsäure, Sulfosalicylsäure, Tanninsäure und Tryptophansäure.

5. Zusammensetzung nach Anspruch 4, wobei die eine oder mehreren Säuren mindestens Citronensäure umfassen.

6. Zusammensetzung nach Anspruch 5, wobei die Menge der Verbindung der Formel 1 0,01 mmol bis 0,3 mmol pro ml der Zusammensetzung beträgt.

7. Zusammensetzung nach Anspruch 6, wobei die eine oder mehreren Säuren Citronensäure und Salzsäure sind, wobei die Salzsäure in einer derart ausreichenden Menge, dass ein pH-Wert der Zusammensetzung von 4,5 bis 6,5 erreicht wird, vorhanden ist.

8. Zusammensetzung nach den Ansprüchen 1, 2, oder 3, wobei Propylenglykol in einer Menge von 40 bis 75 Gew.-%, bezogen auf das Gesamtvolumen, vorhanden ist.

9. Zusammensetzung nach Anspruch 8, wobei der pH-Wert der Zusammensetzung 4,5 bis 6,0 beträgt.

10. Zusammensetzung nach Anspruch 9, wobei der pH-Wert der Zusammensetzung und die Menge von Propylenglykol aus den Werten innerhalb der in Fig. 1 angegebenen Regionen D, E und F ausgewählt sind.

11. Zusammensetzung nach Anspruch 10, wobei Propylenglykol in einer Menge von 57 bis 75 Gew.-%, bezogen auf das Gesamtvolumen, vorhanden ist.

12. Zusammensetzung nach Anspruch 11, wobei der pH-Wert der Zusammensetzung 4,7 bis 5,6 beträgt.

13. Zusammensetzung nach den Ansprüchen 1, 2 oder 3, die ferner ein oder mehrere Antioxidationsmittel, die in einer Menge von 0,01 mg bis 10 mg pro ml der Zusammensetzung vorhanden sind, umfasst.

14. Zusammensetzung nach den Ansprüchen 1, 2 oder 3, die ferner ein oder mehrere Konservierungsmittel, die in einer Menge von 0,01 mg bis 10 mg pro ml der Zusammensetzung vorhanden sind, umfasst.

15. Verfahren zur Herstellung der Zusammensetzung nach den Ansprüchen 1, 2 oder 3, das die Stufen umfasst:

(a) Zugabe von einer oder mehreren Säuren zu Wasser, wobei die Gesamtmenge der einen oder mehreren Säuren derart ausreichend ist, dass eine Lösung mit einer Säurekonzentration von 0,01 bis 0,3 mmol pro ml der Zusammensetzung gebildet wird;
(b) Zugabe von Propylenglykol in einer derart ausreichenden Menge, dass eine Konzentration von Propylenglykol von 25 bis 75 Gew.-%, bezogen auf das Volumen der Zusammensetzung, gebildet wird, und einer Verbindung der Formel 1 in einer derart ausreichenden Menge, dass eine Konzentration der Verbindung von 0,01 bis 0,3 mmol pro ml der Zusammensetzung gebildet wird, zu der Lösung von Stufe (a); und
(c) Einstellen des pH-Werts der Lösung von Stufe (c) zur Bereitstellung eines pH-Werts der Zusammensetzung von 4,5 bis 6,5.

## Revendications

1. Composition aqueuse, comprenant :

(a) un composé de formule 1

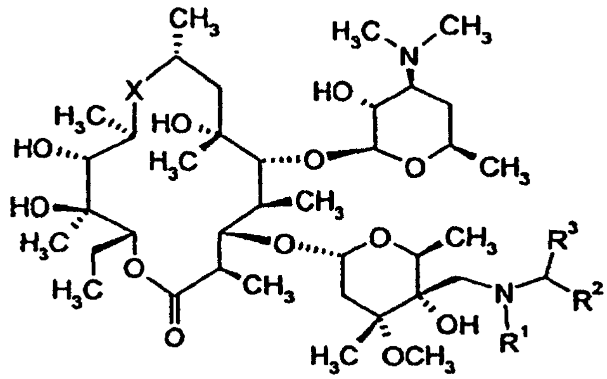

1 ;

(b) un propylène glycol en une quantité d'environ 25 % à environ 75 % en masse par rapport au volume total ;
(c) un ou plusieurs acides pour donner à la composition un pH de 4,5 à 6,5 ; et
(d) de l'eau ;

dans laquelle
$R^1$ et $R^2$ sont tous les deux sélectionnés indépendamment à partir du groupe constitué d'hydrogène, de $(C_1\text{-}C_{10})$alkyle, et de $(C_3\text{-}C_7)$cycloalkyle ;
$R^3$ est choisi à partir du groupe constitué d'hydrogène et de $(C_1\text{-}C_{10})$alkyle ;
X est $-N(R^4)CH_2-$ ou $-CH_2N(R^4)-$ ; et
$R^4$ est $(C_1\text{-}C_3)$alkyle.

**2.** Composition selon la revendication 1, dans laquelle X est $-N(R^4)CH_2-$ et $R^4$ est du méthyle, et dans laquelle $R^2$ est du méthyle.

**3.** Composition selon la revendication 2, dans laquelle soit $R^1$ est du méthyle et $R^3$ est de l'hydrogène, soit $R^1$ est de l'hydrogène et $R^3$ est du méthyle.

**4.** Composition selon les revendications 1, 2 ou 3, dans laquelle les un ou plusieurs acides sont choisis à partir du groupe constitué d'acide acétique, d'acide benzènesulfonique, d'acide citrique, d'acide hydrobromique, d'acide hydrochlorique, d'acide D- et L- lactique, d'acide méthanesulfonique, d'acide phosphorique, d'acide succinique, d'acide sulfurique, d'acide D- et L- tartarique, d'acide p-toluènesulfonique, d'acide adipique, d'acide aspartique, d'acide camphorsulfonique, d'acide 1,2-éthanedisulfonique, d'acide laurylsulfurique, d'acide glucoheptonique, d'acide gluconique, d'acide 3-hydroxy-2-naphthoïque, d'acide 1-hydroxy-2-napthoïque, d'acide 2-hydroxyéthanesulfonique, d'acide malique, d'acide mucique, d'acide nitrique, d'acide napthalènesulfonique, d'acide palmitique, d'acide D-glucarique, d'acide stéarique, d'acide maléique, d'acide malonique, d'acide fumarique, d'acide benzoïque, d'acide cholique, d'acide éthanesulfonique, d'acide glucuronique, d'acide glutamique, d'acide hippurique, d'acide lactobionique, d'acide lysinique, d'acide mandélique, d'acide napadisylique, d'acide nicotinique, d'acide polygalacturonique, d'acide salicylique, d'acide sulfosalicylique, d'acide tannique et d'acide tryptophanique.

**5.** Composition selon la revendication 4, dans laquelle les un ou plusieurs acides comprennent au moins de l'acide citrique.

**6.** Composition selon la revendication 5, dans laquelle la quantité du composé de formule 1 est de 0,01 mmol à 0,3 mmol par mL de la composition.

**7.** Composition selon la revendication 6, dans laquelle les un ou plusieurs acides sont de l'acide citrique et de l'acide hydrochlorique, dans laquelle l'acide hydrochlorique est présent en une quantité suffisante pour donner à la composition un pH de 4,5 à 6,5.

**8.** Composition selon les revendications 1, 2 ou 3, dans laquelle du propylène glycol est présent en une quantité de 40 % à 75 % en masse par rapport au volume total.

**9.** Composition selon la revendication 8, dans laquelle le pH de la composition est de 4,5 à 6,0.

**10.** Composition selon la revendication 9, dans laquelle le pH de la composition et la quantité de propylène glycol sont choisis à partir des valeurs à l'intérieur de régions D, E et F que montre la figure 1.

**11.** Composition selon la revendication 10, dans laquelle du propylène glycol est présent en une quantité de 57 % à 75 % en masse par rapport au volume total.

**12.** Composition selon la revendication 11, dans laquelle le pH de la composition va de 4,7 à 5,6.

**13.** Composition selon les revendications 1, 2, ou 3, qui comprend en outre un ou plusieurs antioxydants présents en une quantité de 0,01 mg à 10 mg par mL de la composition.

**14.** Composition selon les revendications 1, 2, ou 3, qui comprend en outre un ou plusieurs préservatifs présents en une quantité de 0,01 mg à 10 mg par mL de la composition.

**15.** Procédé de préparation de la composition selon les revendications 1, 2, ou 3, comprenant les étapes, dans lesquelles :

(a) on ajoute un ou plusieurs acides à de l'eau, dans lequel la quantité totale des un ou plusieurs acides est suffisante pour produire une solution ayant une concentration en acide de 0,01 à 0,3 mmol par mL de la composition ;
(b) on ajoute à la solution de l'étape (a) du propylène glycol en une quantité suffisante pour produire une concentration de propylène glycol de 25 % à 75 % en masse par rapport au volume de la composition et un composé de la formule 1 en une quantité suffisante pour produire une concentration du composé de 0,01 à 0,3 mmol par mL de la composition ; et
(c) on ajuste le pH de la solution de l'étape (c) pour donner à la composition un pH de 4,5 à 6,5.

FIG. 1

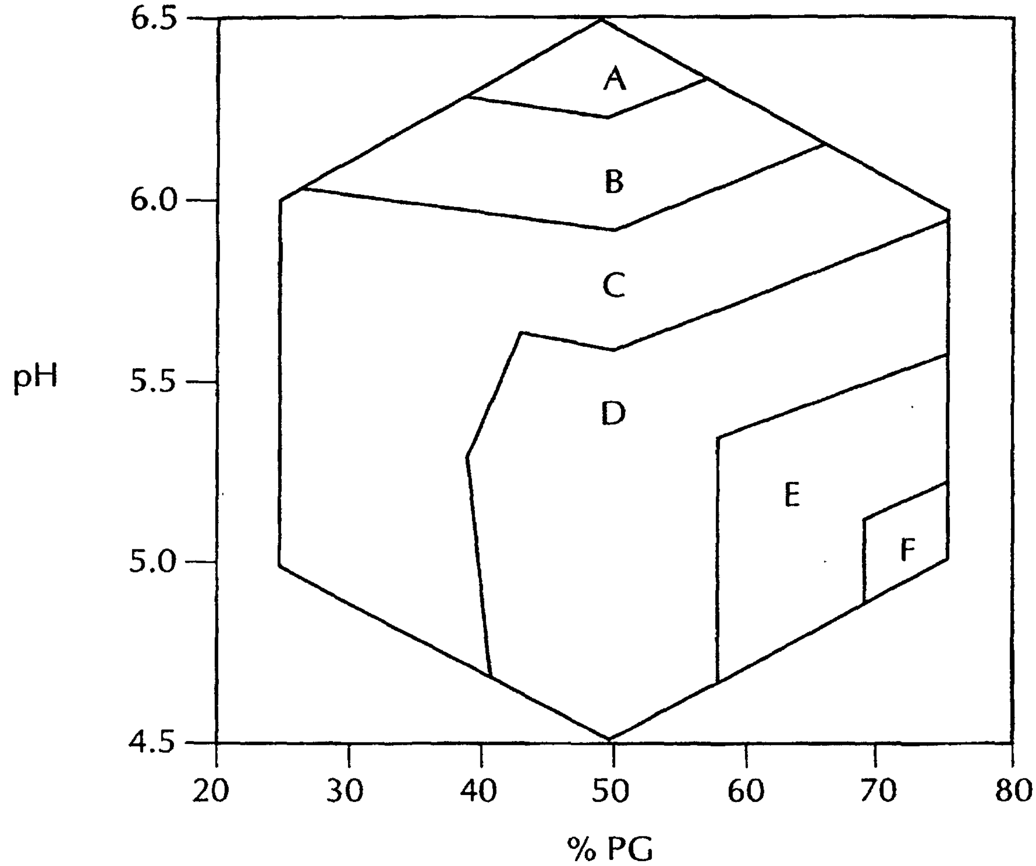


| | | | |
|---|---|---|---|
| Potency (%) | A < 80.0 | B 80-85 | C 85-90 |
| | D 90-95 | E 95-100 | F >100.0 |

Equation A:

z (% potency) = 124.15 + (0.188 x %PG) – (7.633 x pH) - (0.212 x (%PG - 50) x (pH - 5.5)) - (7.967 x $(pH - 5.5)^2$) + (0.003 x $(\%PG - 50)^2$)

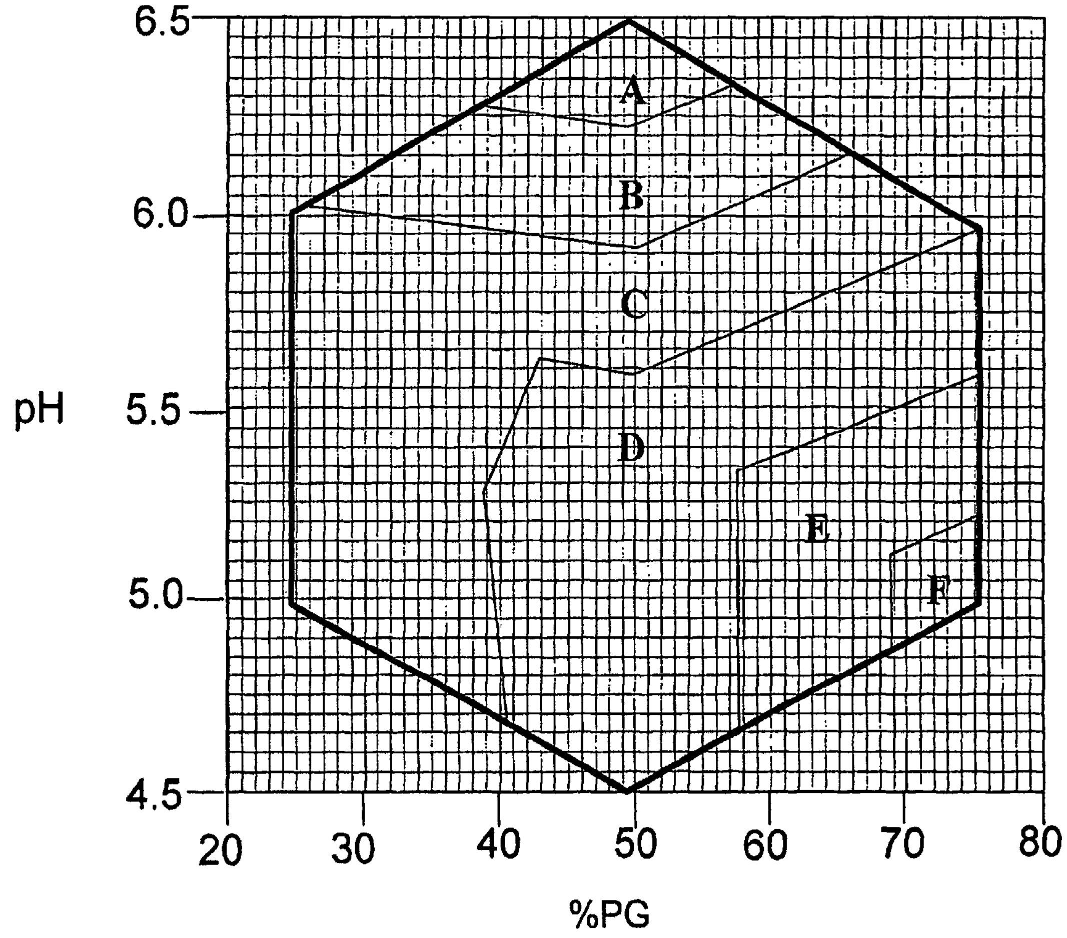


FIG. 1a

Potency (%)

**A** < 80.0 | **B** 80-85 | **C** 85-90
**D** 90-95 | **E** 95-100 | **F** >100.0

FIG. 2

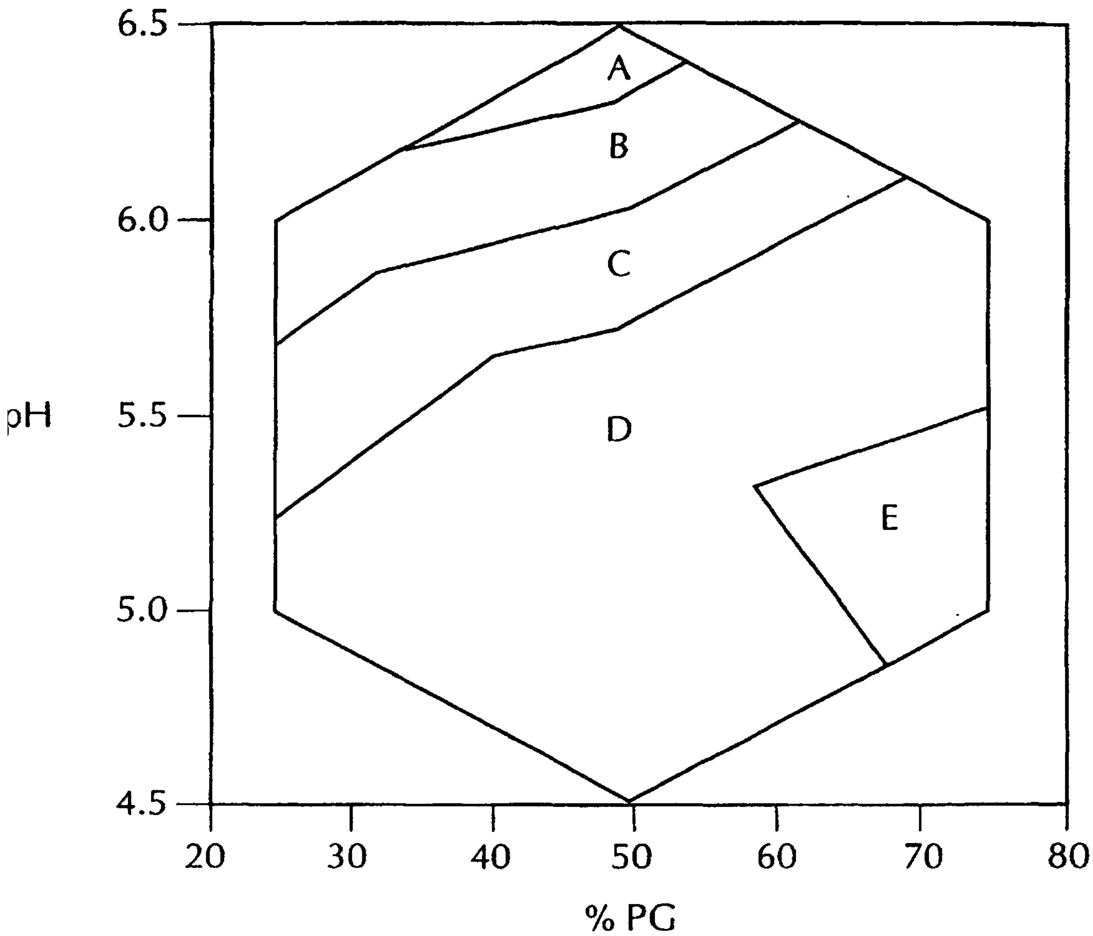


| | | | |
|---|---|---|---|
| Potency (%) | < 80 A | 80-85 B | 85-90 C |
| | | 90-95 D | 95-100 E |

Equation B:

z (% potency) = 126.817 + (0.162 x %PG) – (7.367 x pH) + (0.176 x (%PG-50) x (pH-5.5)) – (10.100 x $(pH-5.5)^2$) – (0.001 x $(\%PG-50)^2$)

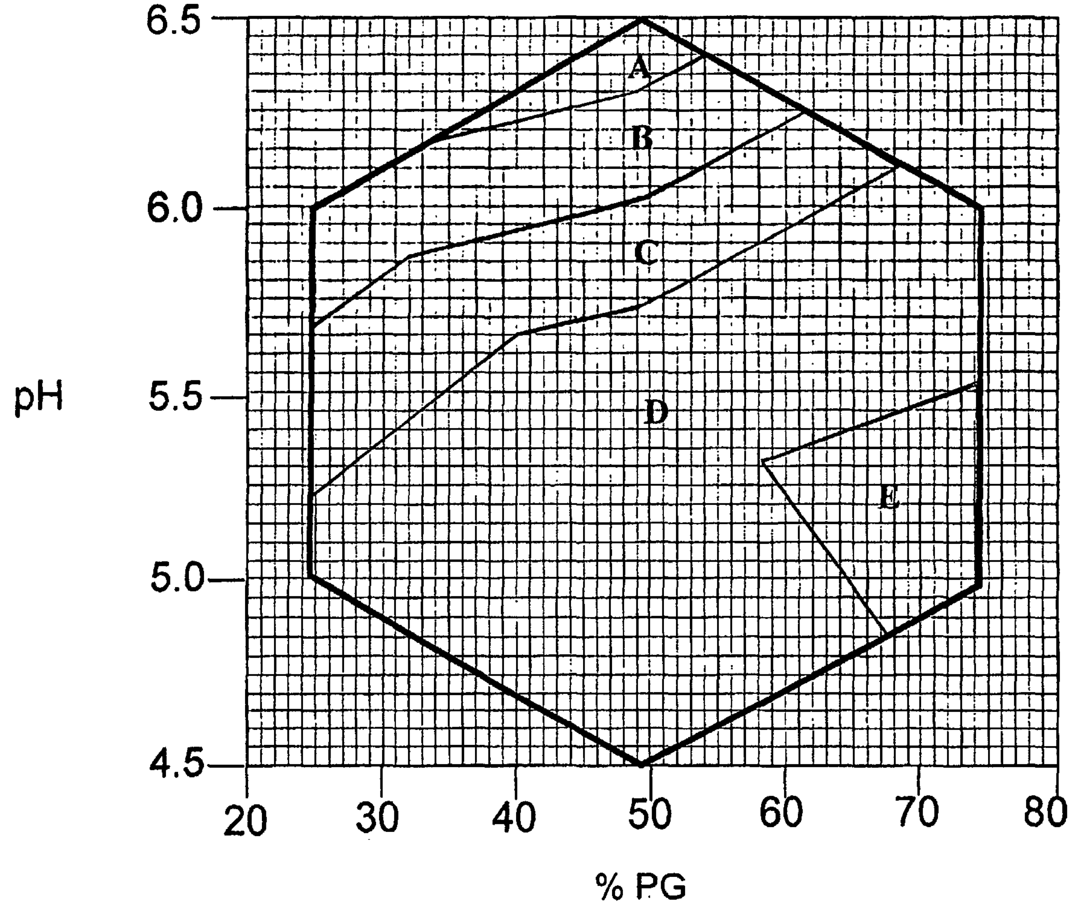


FIG. 2a

Potency (%) A < 80 B 80-85 C 85-90

D 90-95 E 95-100